# EUROPEAN PATENT APPLICATION

(11) **EP 2 149 566 A1**
(43) Date of publication of application: **03.02.2010**
(21) Application number: 08160449.8
(22) Date of filing: 15.07.2008
(51) Int. Cl.: C07D 253/08

(54) **A process for the preparation of telmisartan**

(71) Applicant: Chemo Ibérica, S.A., 08028 Barcelona (ES)
(72) Inventor: Rossi, Renzo, 56127 Pisa (IT); Bellina, Fabio, 56019 Vecchiano fraz. Avane (IT); Cauteruccio, Silvia, 57019 Vicarello (Livorno) (IT); Castaldi, Graziano, Briona N 28072 (IT)
(74) Representative: Barlocci, Anna

(57) **Abstract**

The present invention relates to a process for the preparation of Telmisartan by hydroxycarbonylation of compound of formula (V), 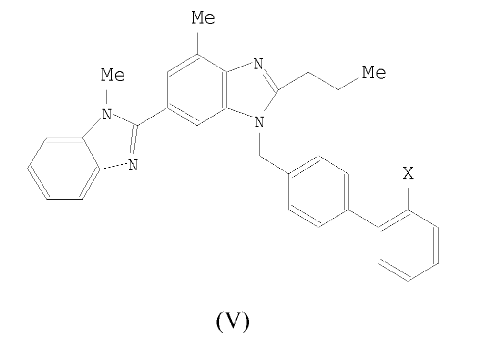 wherein X is a substituent which can be transformed into a carboxy group by hydroxycarbonylation. The invention also relates to intermediates for the synthesis of Telmisartan.

## Description

### Field of the invention

The present invention relates to a process for the preparation of Telmisartan and intermediates for the synthesis thereof.

### Background of the invention

Telmisartan and the physiologically acceptable salts thereof have valuable pharmacological properties. Telmisartan is an angiotensin-II-antagonist which by virtue of its pharmacological properties may be used to treat hypertension and cardiac insufficiency, to treat ischaemic peripheral circulatory disorders, myocardial ischaemia (angina), to prevent the progression of cardiac insufficiency after myocardial infarct, to treat diabetic neuropathy, glaucoma, gastrointestinal diseases and bladder diseases. Telmisartan is also suitable for treating pulmonary diseases, e. g. lung oedema and chronic bronchitis, for preventing arterial restenosis after angioplasty, for preventing thickening of blood vessel walls after vascular operations, and for preventing arteriosclerosis and diabetic angiopathy. In view of the effects of angiotensin on the release of acetyl-choline and dopamine in the brain, Telmisartan is also suitable for alleviating central nervous system disorders, e.g. depression, Alzheimer's disease, Parkinson syndrome, bulimia and disorders of cognitive function.

### Telmisartan is a compound of formula (I)

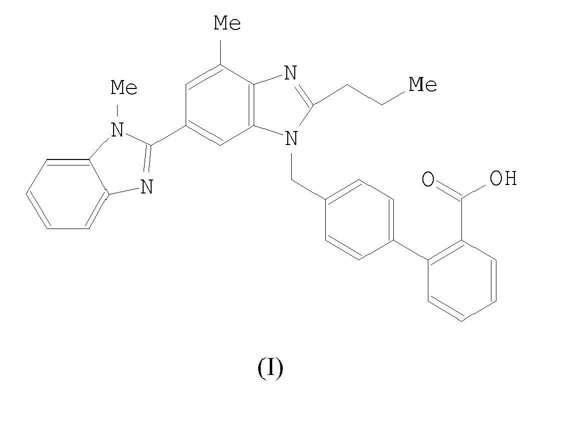

chemically known as 4'-((1,7'-dimethyl-2'-propyl-1H,3'H-2,5,'-bibenzo[d]imidazol-3'-yl)methyl)biphenyl-2-carboxylic acid, which is disclosed in EP 502 314 B1 and marketed under the trade name Micardis®.

Several methods have been used to prepare Telmisartan.

The process described in EP 502 314 B1 comprises the alkylation of 4-methyl-6-(1-methyl-benzimidazol-2-yl)-2-propylbenzimidazole (IV) 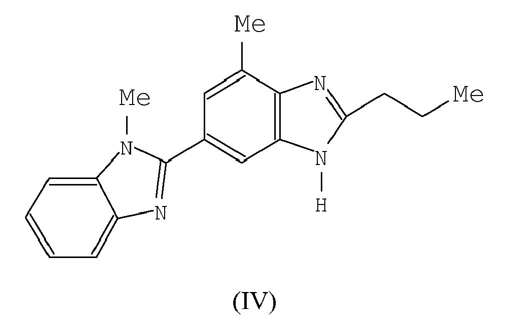 with t-butyl 4'-(bromomethyl)biphenyl-2-carboxylate and subsequently hydrolysis to Telmisartan. t-Butyl 4'-(bromomethyl)biphenyl-2-carboxylate is not commercially available and its synthesis requires a number of steps, among them the protection of the carboxylic function which is finally removed by hydrolysis.

The patent application WO 2006044648 relates to a method for the production of Telmisartan by reacting 4-methyl-6-(1-methyl-benzimidazol-2-yl)-2-propylbenzimidazole (IV) with 4'-bromomethyl-biphenyl-2-carboxylic acid alkyl ester and subsequently hydrolysis.

The patent application WO 2004087676 relates to a method for the production of Telmisartan by reacting 4-methyl-6-(1-methyl-benzimidazol-2-yl)-2-propylbenzimidazole (IV) with 4-bromomethyl-2'-cyanobiphenyl and subsequently conducting hydrolysis of the nitrile to acid function.

The patent application EP 1719766 relates to a method for the production of Telmisartan, by coupling with a Suzuki reaction the *N*-4-bromobenzyl derivative of the compound of formula (IV) with 2-carboxylphenyl boronic acid. As described in EP 1878735, 2-carboxyphenyl boronic acid requires a very laborious process to separate it, since it is extremely soluble in water, making the process unattractive for an industrial application.

Thus, the active substance prepared by the process known up till now can only be obtained in a satisfactory quality after running through a number of process steps. Additional steps of protection and deprotection of the carboxylic function or additional steps to obtain the carboxylic function are often present.

There is therefore the need for an alternative synthesis for the industrial preparation of Telmisartan, which makes use of commercially available or easy to prepare intermediates.

An object of the present invention is to provide an alternative, efficient, economic and commercially useful process for the manufacture of Telmisartan, that avoids the above-identified problems. This invention also relates to intermediates of formula (V) and to a process for preparing them.

### Summary of the invention

It has now been found a process for the preparation of Telmisartan in a straightforward manner exploiting novel intermediates and a hydroxycarbonylation of said intermediates with carbon monoxide.

The method of the present invention involves a process for preparing a compound of formula (I) or a pharmaceutically acceptable salt, hydrate, solvate, or polymorph thereof, 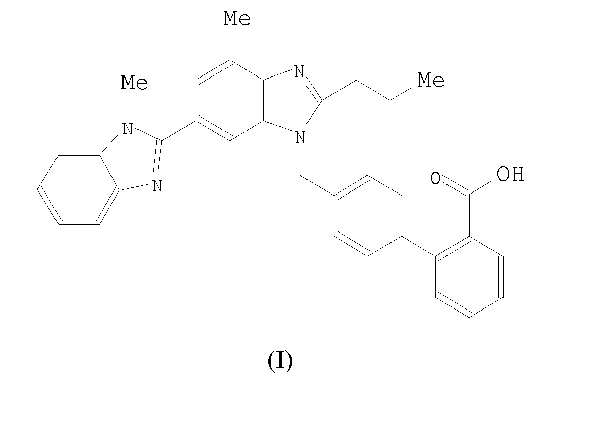 the process comprising hydroxycarbonylation by carbon monoxide of a compound of formula (V) or a salt thereof: 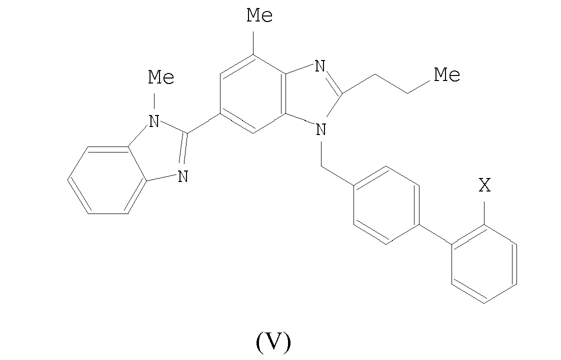 wherein X is a group which may be converted into a carboxy group by hydroxycarbonylation, in the presence of a basic agent, water, and a catalytic system comprising a metal and an organic ligand.

As a further aspect, the invention provides a compound of formula (V), defined as above, or a salt, a hydrate, a solvate thereof.

As an additional aspect, the invention provides a process for preparing a compound of formula (V), comprising the reaction of compound of formula (IV) 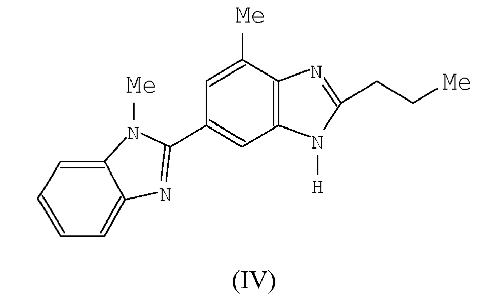 with a compound of formula (III) 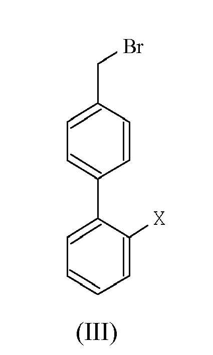 wherein X is a group which may be converted into a carboxy group by hydroxycarbonylation, in a solvent, and optionally in the presence of a base.

In a further aspect, the invention provides the use of compounds of formula (V) as intermediates in the preparation of Telmisartan.

With the process of the present invention the carboxylic function of Telmisartan is straight obtained, without the additional steps to obtain the carboxylic function or without the additional steps of protection and deprotection of the carboxylic function. The present method makes use of generally inexpensive and readily available reagents.

### Detailed description of the invention

All terms as used herein in this application, unless otherwise stated, shall be understood in their ordinary meaning as known in the art. Other more specific definitions for certain terms as used in the present application are as set forth below and are intended to apply uniformly through-out the specification and claims unless an otherwise expressly set out definition provides a broader definition.

The term "C₁-C₃ alkyl" refers to a straight or branched hydrocarbon having from 1 to 3 carbon atoms. Examples of alkyl groups include, without limitation, methyl, ethyl, *n-*propyl, isopropyl, and the like.

The term "C₁-C₈ alkyl" refers to a straight or branched hydrocarbon having from 1 to 8 carbon atoms, preferably 1 to 4 carbon atoms, more preferably up to 2 carbon atoms. Examples of alkyl groups include, without limitation, methyl, ethyl, *n*-propyl, isopropyl, *n-*butyl, *sec*-butyl, isobutyl, *tert*-butyl, *n*-pentyl, *n*-hexyl, *n*-heptyl, *n*-octyl groups and the like.

The term "aryl" refers to an aromatic carbocyclic group having a single ring (e.g., phenyl), multiple rings (e. g., biphenyl), or multiple condensed rings in which at least one is aromatic (*e.g.*, 1,2,3,4-tetrahydronaphthyl, 1-naphthyl, 2-naphthyl, anthryl, or phenanthryl). The aryl group may be optionally substituted. A preferable aryl group of the present invention is phenyl, p-tolyl.

The term "carboxy" refers to the -COOH group.

The term "halogen" refers to chlorine, bromine, fluoride or iodine.

The term "group which may be converted into a carboxy group by hydroxycarbonylation" refers, for example, to halogens, to a hydroxyl group activated by esterification with an alkyl or arylsulfonyl group, such as methanesulfonyl, trifluoromethanesulfonyl, toluenesulfonyl, benzenesulfonyl and the like.

The term "substituted" refers to groups substituted with from 1 to 5 substituents selected from the group consisting of C₁-C₈-alkyl, halogen, amino, aminosulfonyl, aminocarbonyl, sulfinyl, sulfanyl, sulfonyl, hydroxy, acyl, alkoxy, alkoxycarbonyl, carbamate, trihalomethyl, cyano, mercapto, nitro, and the like.

The term "about" encompasses the range of experimental error that may typically occur in a measurement.

The term "pharmaceutically acceptable salt(s)", as used herein, unless otherwise indicated, refers to the relatively non-toxic inorganic and organic acid-addition salts and the base-addition salts, of the compounds of the present invention. These salts may be prepared in situ during the final isolation and purification of the compounds.

In particular, the basic-addition salts may be prepared by separately reacting the compound with an organic or inorganic base and isolating the salt thus formed. The resulting salts are, for example, metal salts, particularly alkali metal salts, alkaline-earth metal salts and transition metal salts (such as sodium, potassium, calcium, magnesium, aluminum), or salts obtained with bases, such as ammonia or secondary or tertiary amines (such as diethylamine, triethylamine, *tert*-butylamine, piperidine, piperazine, morpholine), or with basic amino-acids, for example lysine, or with osamines (such as meglumine), or with amino alcohols (such as 3-aminobutanol and 2-aminoethanol).

The invention also relates to pharmaceutically acceptable salts with organic or inorganic acids. In particular, the acid-addition salts may be prepared by separately reacting the compound with an organic or inorganic acid and isolating the salt thus formed. The resulting salts are, for example, hydrochloride, hydrobromide, hydroiodide, nitrate, sulfate, bisulfate, phosphate, dihydrogenophosphate, acid phosphate, isonicotinate, acetate, trifluoroacetate, lactate, salicylate, citrate, acid citrate, tartrate, pantothenate, bitartrate, ascorbate, succinate, maleate, gentisinate, fumarate, gluconate, glucuronate, saccharate, formate, benzoate, glutamate, methanesulfonate, ethanesulfonate, benzenesulfonate, p-toluenesulfonate, 2-naphtalenesulfonates, camphorsulfate and pamoate [i.e., 1,1'-methylene-bis-(2-hydroxy-3-naphthoate)] salts.

In addition to the pharmaceutically acceptable salts, other salts are included in the invention. They may serve as intermediates in the purification of the compounds, in the preparation of other salts, or in the identification and characterization of the compounds or intermediates.

The pharmaceutically acceptable salts of compounds of formula (I) or (V) may exist as solvates, such as with water, methanol, ethanol, dimethylformamide, ethyl acetate, and the like. Mixtures of such solvates can also be prepared. The source of such solvate can be from the solvent of crystallization, inherent in the solvent preparation or crystallization, or adventitious to such solvent. Such solvates are within the scope of this invention.

The term "hydrate" refers to a solvate comprising a disclosed or claimed compound and a stoichiometric or non-stoichiometric amount of water.

The term "solvate" refers to a molecular complex comprising a disclosed or claimed compound and a stoichiometric or non-stoichiometric amount of one or more solvent molecules (*e.g.*, ethanol).

Generally, the chemical transformations described throughout the specification may be carried out using substantially stoichiometric amounts of reactants, though certain reactions may benefit from using an excess of one or more of the reactants. Additionally, many of the reactions disclosed throughout the specification, may be carried out at room temperature, but particular reactions may require the use of higher or lower temperatures, depending on reaction kinetics, yields, and the like. Furthermore, many of the chemical transformations may employ one or more compatible solvents, which may influence the reaction rate and yield. Depending on the nature of the reactants, the one or more solvents may be polar protic solvents, polar aprotic solvents, non-polar solvents, or some combination.

The compounds obtained by the chemical transformations of the present invention, can be used for the following steps without further purification or can be effectively separated and purified by employing a conventional method well known to those skilled in the art, such as recrystallization, column chromatography, or by transforming then into a salt or by washing with an organic solvent or with an aqueous solution, eventually adjusting pH.

The process of the invention for making Telmisartan (I) is illustrated in the following reaction Scheme 1. 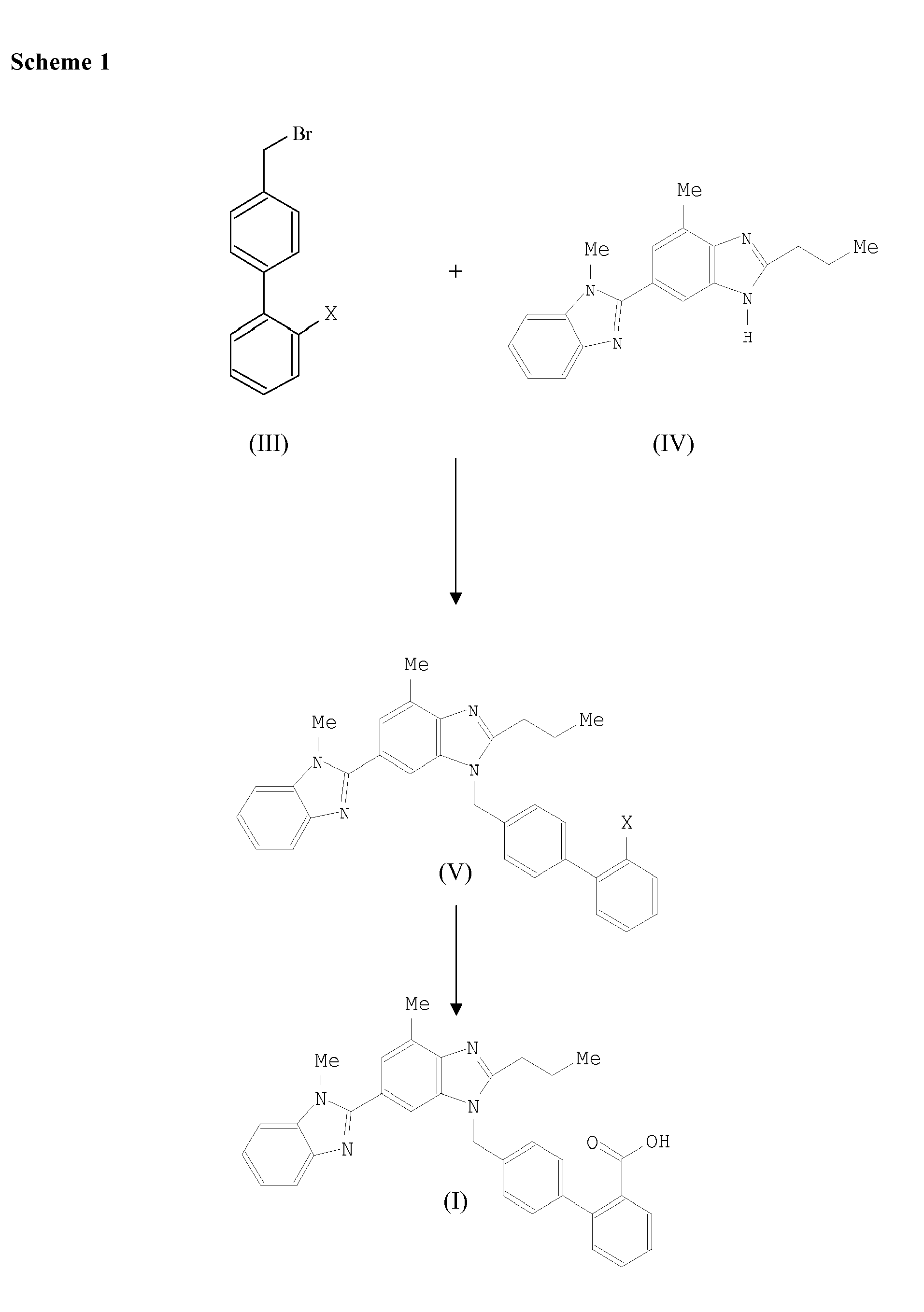 wherein X in compounds of formula (III) or (V) is a group which may be converted into a carboxy group by hydroxycarbonylation. For example, leaving groups, such as a halogen; -OSO₂R, where R is a C₁-C₈ alkyl group, CF₃, or an aryl group optionally substituted, may be converted into a carboxy group by hydroxycarbonylation. Preferably, X is bromine, -OSO₂Methy, -OSO₂CF₃, -OSO₂(p-tolyl); more preferably X is bromine. In a first aspect, the invention provides a process for preparing a compound of formula (I) or a pharmaceutically acceptable salt, hydrate, solvate, or polymorph thereof, the process comprising hydroxycarbonylation by carbon monoxide of a compound of formula (V) or a salt thereof, as defined above, in the presence of a basic agent, water, and a catalytic system comprising a metal and an organic ligand. (Scheme 1).

According to the invention, for the hydroxycarbonylation reaction, the technical gas carbon monoxide may be applied at a pressure ranging from atmospheric pressure to 50 psi, the total pressure can vary within wide limits. Preferably, the pressure is atmospheric pressure.

Carbon monoxide may be also generated *in situ*. Any internal condensed source of carbon monoxide known to those skilled in the art can be used. For example, carbon monoxide may be generated *in situ* by the treatment of formate salts, HCOOM, wherein M is an alkali metal or alkaline earth metal, preferably lithium, sodium or potassium, with Ac₂O, in the presence of a base, such as *i*-Pr₂EtN, Et₃N. Cacchi, S. et al. Org. Lett. 2003, 5, 4269-4272 have reported on the use of acetic acid anhydride and lithium formate as starting materials for carbon monoxide.

According to the invention, the reaction is catalyzed by a catalytic system comprising a metal and an organic ligand. The components of the catalytic system may be added separately to the reaction mixture. The organic ligand complexes the metal and forms the active metal-ligand complex catalyst. Preferably, the catalytic system comprises a transition metal selected from Pd or Ru, more preferably Pd Also preferably, Pd is added to the reaction mixture as Pd(OAc)₂ or PdCl₂. The amount of metal to be employed can vary within wide limits. In general, an amount of between 0.1% moles and 5 % moles with respect to substrate gives satisfactory results. Preferably, the concentration of Pd is between 1% molar and 2% molar with respect to substrate.

According to the invention, the organic ligand comprises, for example, a phosphine, such as triarylphosphine, preferably P(Ph)₃; trialkylphosphine, preferably PCy₃ (where Cy is cyclohexyl); or a bidendate diphosphine ligand, comprising xanthene-based diphosphines (Xantphos ligands), such as 9,9-dimethyl-4,5-bis(diphenylphosphino)xanthene (Xantphos), 2,7-di-tert-butyl-9,9-dimethyl-4,5-bis(diphenylphosphino)xanthene (tBu-Xantphos); phenoxazine-based diphosphines (Nixantphos ligands), such as 4,6-bis(diphenylphosphino)phenoxazine; (oxydi-2,1-phenylene)bis(diphenylphosphine) (DPEphos); 1,1'-bis(diphenylphosphino)ferrocene (dppf). Preferably, the organic ligand is triphenylphosphine.

The molar amount of the organic ligand may be comprised from 2% molar to 10% molar with respect to substrate, preferably from 8 to 10% molar with respect to substrate.

The metal/organic ligand molar ratio is advantageously comprised from 1:1 to 1:10, preferably 1:5.

According to the invention, a basic agent can be an organic base, for example straight or branched tertiary amines, such as triethylamine, diisopropyl ethyl amine, tetramethylethylenediamine, tributylamine, pyridine, N-methylmorpholine, tetramethylurea, methylpyrrolidinone, 4-dimethylaminopyridine, proton sponge or dimethylaniline; or an inorganic base, for example alkali or alkali earth hydroxides, such as sodium hydroxide, calcium hydroxide, or alkali or alkaline-earth metal carbonates, such as K₂CO₃, or hydrated Cesium salts, such as CsOAc. Preferably, a basic agent is CsOAc, Bu₃N, K₂CO₃, more preferably CsOAc.

The hydroxycarbonylation reaction can be carried out in a suitable solvent. As stated above, the hydroxycarbonylation reaction of a compound of formula (V) is carried out in the presence of water. Generally, the reaction is carried out in a miscible mixture of water and polar aprotic solvents. Suitable solvents are ethers, such as tetrahydrofuran, 2-methyltetrahydrofuran or 1,4-dioxane; polar aprotic solvents, such as dimethylformamide, dimethylacetamide or N-methylpyrrolidone; or miscible mixtures thereof. Preferably, the solvent is a mixture of dimethylformamide/water.

The reaction may be carried out over a wide range of temperatures to achieve the desired reaction rate. The temperature is generally between room temperature and reflux temperature, preferably between 110 and 130 °C.

According to a particularly preferred embodiment of the invention, the reaction is carried out in a dimethylformamide/water mixture, with a compound of formula (V), wherein X is a bromine atom. Telmisartan may be recovered by method known in the art, such as filtration, crystallization, salification, centrifugation, etc. The conversion of a compound of formula (I) into a respective salt or the conversion of a salt of a compound of formula (I) to the unsalified form can be accomplished according to known methods.

The hydroxycarbonylation reaction according to the invention applies to a variety of substrates, including different biphenyl derivatives, and allows, for instance, the efficient preparation with high yields of biphenyl carboxylic acids of formula (VI), 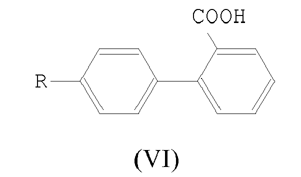 wherein R is H, or a C₁-C₃ alkyl group, otherwise difficult to prepare.

The compounds of formula (V) are novel compounds and are also an object of the present invention.

In a further aspect, the present invention provides a compound of the general formula (V), defined as above, as well as its salts, hydrates, solvates thereof.

The compounds of formula (V) wherein X is an halogen atom, in particular bromine, are preferred.

In an additional aspect, the present invention provides a process for preparing a compound of formula (V), comprising the reaction of the commercially available (1,4'-dimethyl-2'-propyl[2,5'-bis-1H-benzimidazol]-3'-yl)methyl (IV) with a compound of formula (III), wherein X is defined as above, preferably X is a bromine atom, in a suitable solvent and optionally in the presence of a base. A solvent may be an aliphatic or aromatic hydrocarbon, such as hexane or heptane, toluene, benzene or xylenes; an ether, such as tetrahydrofuran or dioxane; an ester, such as ethyl acetate, isopropyl acetate or butyl acetate; a chlorinated solvent, such as dichloromethane; a polar aprotic solvent, such as dimethylformamide, dimethylacetamide, N-methyl pyrrolidone, dimethylsulfoxide; or mixtures thereof. Preferably, the solvent is dimethylacetamide (DMA).

A base can be an organic or inorganic base, such as sodium carbonate, potassium carbonate, sodium hydroxide, potassium hydroxide, sodium methoxide, potassium methoxide, potassium tert-pentoxide, potassium tert-butoxide, potassium n-butoxide, sodium hydride, triethylamine, or pyridine, while the latter two may also be used as solvents, preferably potassium tert-butoxide.

According to the invention, the reaction can be carried out at a temperature ranging from about 0°C to the reflux temperature of the reaction mixture, preferably from about 5°C to 30°C.

In a preferred embodiment, a base was added portionwise to a solution of the compound (IV) in DMA at a temperature between 0°C and 30°C and then a solution of compound of formula (III) in DMA was added portionwise at a temperature below 5°C. Preferably, the ratio base/(IV) is of about 1 to 1.2 mol/mol. The stirring was maintained for two hours at a temperature between 5-10°C and the desired product (V) was recovered by extraction with a suitable solvent and resuspension in a solvent, such as diethyl ether.

Compounds of formula (V) are intermediates for the preparation of pharmaceutically effective Telmisartan.

In a further aspect, the present invention provides use of compounds of formula (V), defined as above, as intermediates in the preparation of Telmisartan.

The compounds useful according to the invention may be prepared, unless specifically specified, by the application or adaptation of known methods, by which are meant methods used heretofore or described in the literature, patents or patent applications, the Chemical Abstracts and on the Internet.

Compounds of formula (III), wherein X is defined as above, can be prepared starting from compounds of formula (II), 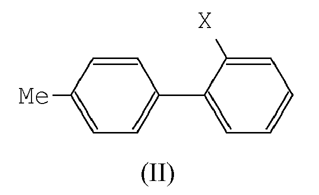 wherein X is defined as above, according to methods well known to the persons skilled in the art.

In general, the synthesis pathways for any individual compound of formula (II) and formula (III) will depend on the specific substituents of each molecule and upon the ready availability of intermediates necessary; again such factors being appreciated by those of ordinary skill in the art.

Depending on the nature of X different synthetic strategies may be selected for the synthesis of compounds of formula (III). For instance, compounds of formula (III), wherein X is Br (IIIa) may be prepared by reaction of (II) with a radical initiator, such as dibenzoyl peroxide, di-*tert*-butylperoxide, and NBS in a chlorinated hydrocarbon, such as CH₂Cl₂, CCl₄. An example of this conversion is described by Djerassi, C. Chem. Rev. 1948, 43, 271.

Compounds of formula (II) may be prepared by the application of the Suzuki reaction (Miyaura, N. et al. Tetrahedron Letters 1979, 3437; Miyaura, N.; Suzuki, A. Chem. Commun. 1979, 866).

Many of the compounds described herein, are capable of forming pharmaceutically acceptable salts. These salts include, without limitation, base salts.

One may prepare a pharmaceutically acceptable base salt by contacting a compound's free acid or zwitterion with a sufficient amount of a desired base to produce a nontoxic salt. If the salt precipitates from solution, it may be isolated by filtration; otherwise, the salt may be recovered by evaporating the solvent.

One may also regenerate the free base by contacting the acid addition salt with a base (or the base salt with an acid). Though certain physical properties of the free base (or free acid) and its respective acid addition salt (or base salt) may differ (*e.g.*, solubility, crystal structure, hygroscopicity, etc. ), a compound's free base and acid addition salt (or its free acid and base salt) are otherwise the same for purposes of this disclosure.

As mentioned above the compound of the invention has useful therapeutic properties. It is contemplated that the compounds of the present method can be found or isolated in the form of hydrates or solvates, in different polymorphic forms, i.e., different crystalline forms, which are considered to fall within the scope of the present invention.

While the present invention has been described in terms of its specific embodiments, certain modifications and equivalents will be apparent to those skilled in the art and are included within the scope of the present invention.

In the following, the present invention shall be illustrated by means of some examples, which are not construed to be viewed as limiting the scope of the invention.

### Examples

The following abbreviations refer respectively to the definitions below:
DMA (N,N-dimethylacetamide); DMF (dimethylformamide); GLC (Gas Liquid Chromatography); hr (hour); hrs (hours); MPLC (Medium Pressure Liquid Chromatography); Pd₂(dba)₃ (tris(dibenzylideneacetone)dipalladium(0)).

### Example 1

### 2-Bromo-4'-methylbiphenyl (IIa)

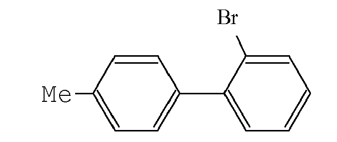

In a 100 mL glass vessel, 1-bromo-2-iodobenzene (1.98 g, 7 mmol), p-tolylboronic acid (1.05 g, 7.7 mmol) and anhydrous toluene (19.8 mL) were mixed. An aqueous solution of K₂CO₃ (8.33 mL, 2M) was added and nitrogen was bubbled in the mixture for 30 minutes. Pd₂(dba)₃ (0.0962 g, 0.105 mmol) and PPh₃ (0.220 g, 0.84 mmol) were added under nitrogen atmosphere and the mixture was stirred at 90°C for 26 hrs. The reaction was monitored by GLC (capillary column AT-5, 30 m). The mixture was quenched at room temperature in water (100 mL) and extracted with diethyl ether. The combined organic layer was washed with brine, dried and concentrated under reduced pressure to a yellow oil. Ligroin (25 mL) was added and the organic phase is filtered through Celite®, then evaporated under reduced pressure to a residue, which was purified by silica gel MPLC (Merck silica gel 60, particle size 0.015-0.040 mm) eluting with Ligroin, yielding the title compound (IIa) (1.43 g) as a colourless oil.
Yield: 83%.
¹H NMR (200 MHz, CDCl₃): δ 7.64 (d, 1H; J=7.6 Hz), 7.23 (m, 7H), 2.39 (s, 3H) ¹³C NMR (50 MHz, CDCl₃): δ 142.6, 138.2, 137.3, 133.1, 131.3, 129.2 (2C), 128.7 (2C), 128.5, 127.3, 122.7, 21.2
EIMS (*m*/*z*): 249 (14), 248 (96), 247 (21), 246 (100), 167 (31), 166 (24), 165 (72), 152 (34), 82 (10)

### Example 2

### 2-Bromo-4'-(bromomethyl)biphenyl (IIIa)

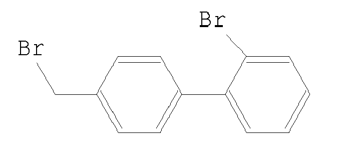

To a solution of 2-bromo-4'-methylbiphenyl (IIa) (2.061 g, 8.35 mmol), in anhydrous CCl₄ (50 mL), freshly recrystallized N-bromo-succinimide (1.486 g, 8.35 mmol) and benzoyl peroxide (0.020 g, 0.0835 mmol) were added, under nitrogen atmosphere. The mixture was heated under reflux temperature for 3 hrs. A white solid precipitated. The reaction was monitored by GLC (capillary column AT-5, 30 m). The mixture was filtered at room temperature and the white solid washed with CCl₄. The reunited organic phases were concentrated under reduced pressure to a residue, that was washed with Ligroin (40 mL) for about 1 hr. The resulting mixture was filtered and evaporated to the title compound (IIIa) (2.93 g), as a yellow oil, which was used for further reactions without purification.

### Example 3

### 3'-((2'-bromobiphenyl-4-yl)methyl)-1,7'-dimethyl-2'-propyl-1H,3H-2,5'-bibenzo[d]imidazole (Va)

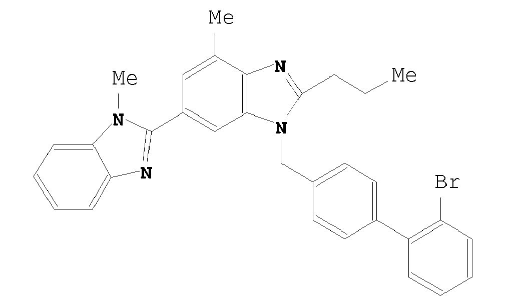

A mixture of 4-methyl-6-(1-methyl-benzimidazol-2-yl)-2-propylbenzimidazole (IV) (1.49 g, 4.60 mmol) and DMA (6 mL) was stirred until a clear solution was obtained. t-BuOK (0.569 g, 5.067 mmol) was added portionwise, keeping the temperature below 30°C. The resulting orange mixture was stirred at room temperature for 1 h, then cooled to 0°C. A solution of 77% chemically pure 2-bromo-4'-bromomethylbiphenyl (IIIa) (2.88 g, 6.91 mmol of chemically pure (IIIa)) in DMA (3 mL) was added dropwise, maintaining the temperature below 5°C. The resulting mixture was stirred for 2 h at 5-10°C and then poured into a mixture of toluene (20 mL) and water (20 mL) and warmed up to 75°C. The phases were separated and the aqueous phase was extracted with toluene (3 x 20 mL) at 50°C and with CH₂Cl₂ (15 mL) at room temperature. The collected organic phases were concentrated under reduced pressure and the resulting yellow oil was treated with Et₂O (100 mL). The resulting mixture was stirred for 8 h at room temperature and the solid which was so obtained was filtered and dried under reduced pressure to give the title crude compound (Va) (0.6565 g), that after recrystallization from CH₂Cl₂ and petroleum ether gave the pure title compound (Va) (0.5241 g).

The pure title compound (Va) (1.3845 g) was also obtained, as a light yellow solid, by cooling the ether filtrate to 0°C, filtering and driying the solid so obtained under reduced pressure.
Overall yield: 75.4%.
¹H NMR (200 MHz, DMSO-d6): δ7.90-6.95 (m, 4H), 5.66 (s, 2H), 3.83 (s, 3H), 2.93 (t, 2H, *J* = 7.4 Hz), 2.64 (s, 3H), 1.80 (m, 2H), 0.99 (t, 3H, *J* = 7.0 Hz).
¹³C NMR (50.3 MHz, DMSO-d6): δ 156.1, 154.0, 142.6, 142.5, 141.3, 139.6, 136.6, 136.1, 134.7, 132.9, 131.3, 129.5 (2C), 129.3, 128.2, 127.9, 126.3 (2C), 123.3, 123.1, 121.9, 121.6, 118.6, 110.3, 109.1, 97.1, 46.0, 33.1, 28.7, 20.6, 16.4, 13.7.

### Example 4

### 4'-((1,7'-dimethyl-2'-propyl-1H,3'H-2,5,'-bibenzo[d]imidazo1-3'-yl)methyl)biphenyl-2-carboxylic acid - Telmisartan (I)

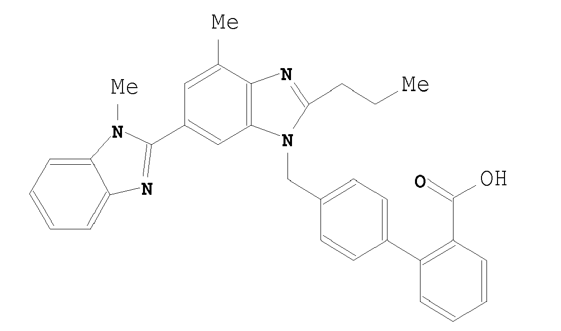

Under CO atmosphere (1 atm) and magnetic stirring, Pd(OAc)₂ (0.0045 g, 0.02 mmol) and P(Ph)₃ (0.0262 g, 0.1 mmol) were rapidly added to a solution of 3'-((2'-bromobiphenyl-4-yl)methyl)-1,7'-dimethyl-2'-propyl-1*H*,3*H*-2,5'-bibenzo[*d*]imidazole (Va) (0.5495 g, 1 mmol), CsOAc (0.2552 g, 1.33 mmol) in DMF (1.07 mL), water (0.0820 mL) and Bu₃N (0.3707 g, 2 mmol, 0.4765 mL). The mixture was heated at 120 °C for 24 hrs, then cooled to room temperature, diluted with CH₂Cl₂ (20 mL) and poured out in a solution of NaOH (10%, 100 mL). The mixture was stirred for 40 minutes, then the aqueous phase was extracted 4 times with CH₂Cl₂, dried over Na₂SO₄ and evaporated to an oil, which was suspended in diethyl ether (100 mL). A precipitate was formed and filtered, yielding the title compound (I) (0.5943 g) as a solid. This solid was suspended in 30% NH₄OH (8 mL) and acetone (5 mL) and the mixture was stirred at room temperature for 0.5 h and filtered. The yellow filtrate was acidified (pH 5) using glacial AcOH and the resulting solution was concentrated under reduced pressure. The resulting solid was treated with water (20 mL) and the mixture, after stirring for 0.5 h, was filtered. The collected solid was dried under reduced pressure to give the title compound (I) (0.309 g), as a pale yellow solid.
Yield: 60%.
¹H NMR (300 MHz, DMSO-d6): δ 7.65-7.70 (m, 3H), 7.40-7.56 (m, 4H), 7.15-7.32 (m, 7H), 5.60 (s, 2H), 3.80 (s, 3H), 2.91 (m, 2H), 2.61 (s, 3H), 1.80 (m, 2H), 0.98 (m, 2H).
¹³C NMR (75 MHz, DMSO-d6): δ 169.50. 156.19, 154.01, 142.70. 142.35, 140.48, 140.16, 136.60. 135.90. 134.70. 132.29, 130.80. 130.32, 129.08, 128.68, 128.21, 127.28, 126.37, 123.14, 122.06, 121.80. 118.65, 110.37, 109.28, 46.12, 31.74, 28.80. 20.71, 16.43, 13.81

## Claims

1. A process for preparing a compound of formula (I) or a pharmaceutically acceptable salt, hydrate, solvate, or polymorph thereof: 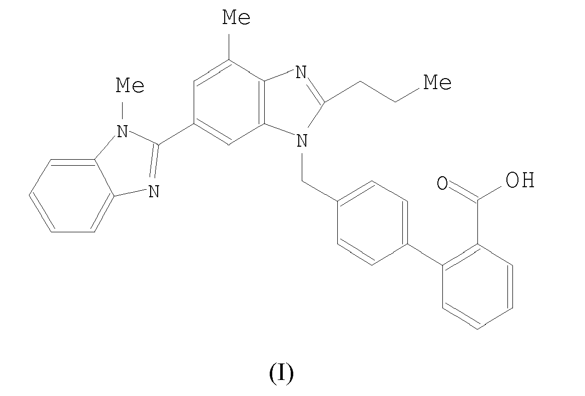 the process comprising hydroxycarbonylation by carbon monoxide of a compound of formula (V) or a salt thereof: 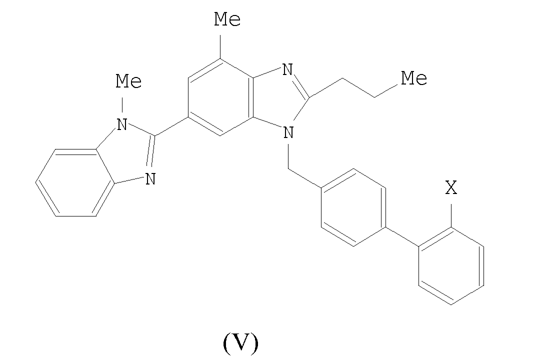 wherein X is a group which may be converted into a carboxy group by hydroxycarbonylation, in the presence of a basic agent, water, and a catalytic system comprising a metal and an organic ligand.

2. The process according to claim 1, wherein X is:
halogen;
-OSO₂R, where R is a C₁-C₈ alkyl group, CF₃, or an aryl group optionally substituted.

3. The process according to claim 2, wherein X is bromine.

4. The process according to claim 1, wherein the carbon monoxide is at an atmospheric pressure or is generated *in situ*.

5. The process according to claim 1, wherein the metal is a transition metal.

6. The process according to claim 5, wherein the transition metal is added to the reaction system as palladium (II) acetate or palladium (II) chloride.

7. The process according to claim 1, wherein the organic ligand is a phosphine.

8. The process according to claim 7, wherein said phosphine is triphenylphosphine, or a bidendate diphosphine ligand, comprising Xantphos ligands, Nixantphos ligands, (oxydi-2,1-phenylene)bis(diphenylphosphine) (DPEphos), or 1,1'-bis(diphenylphosphino)ferrocene (dppf).

9. The process according to claim 8, wherein said phosphine is triphenylphosphine.

10. The process according to claim 1, wherein the basic agent is CsOAc.

11. The process according to claim 1, wherein the hydroxycarbonylation is conducted in a mixture of dimethylformamide/water.

12. A compound of formula (V), or a salt, a hydrate, a solvate thereof, 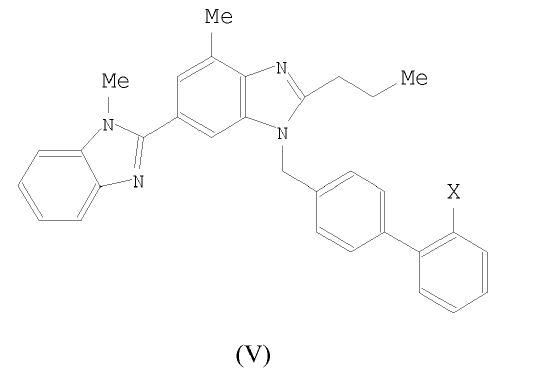 wherein X is a group which may be converted into a carboxy group by hydroxycarbonylation.

13. A compound according to claim 12, wherein X is:
halogen;
-OSO₂R, where R is a C₁-C₈ alkyl group, CF₃, or an aryl group optionally substituted.

14. A compound according to claim 13, wherein X is bromine.

15. A process for preparing a compound according to claim 12, comprising the reaction of compound of formula (IV) 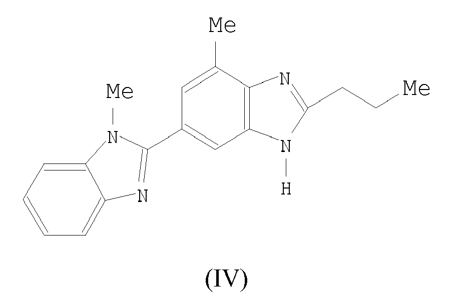 with a compound of formula (III) 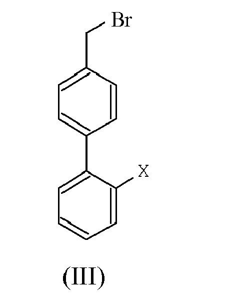 wherein X is a group as defined in claims 12 or 13,
in a solvent and optionally in the presence of a base.

16. Use of compounds of formula (V), as claimed in claim 12, as intermediates in the preparation of Telmisartan.
